# EUROPEAN PATENT SPECIFICATION

(11) **EP 3 154 552 B1**
(45) Date of publication and mention of the grant of the patent: **24.03.2021**
(21) Application number: 15807515.0
(22) Date of filing: 10.06.2015
(51) Int. Cl.: A61K 9/70, A61K 31/737, A61K 47/20, A61K 47/36

(54) **DEVICE AND METHOD TO TREAT OR PREVENT JOINT DEGENERATION**
VORRICHTUNG UND VERFAHREN ZUR BEHANDLUNG ODER PRÄVENTION VON GELENKVERSCHLEISS
DISPOSITIF ET MÉTHODE POUR TRAITER OU PRÉVENIR LA DÉGÉNÉRESCENCE ARTICULAIRE

(30) Priority: 11.06.2014 AU 2014902219
(43) Date of publication of application: 19.04.2017
(73) Proprietor: International Scientific Pty Ltd, Leederville, Western Australia 6007 (AU)
(72) Inventor: EDWARDS, Jeffrey D., Nedlands, Western Australia 6009 (AU); MCILDOWIE, Matthew, Craigie, Western Australia 6025 (AU); MOURSOUNIDIS, John, Mount Claremont, Western Australia 6010 (AU)
(74) Representative: Potter Clarkson
(86) International application number: PCT/AU2015/000349
(87) International publication number: WO 2015/188223

(56) References cited:
- WO-A2-01/76553
- KR-B1- 101 338 516
- RO-A2- 128 699
- US-A1- 2013 144 109
- US-A1- 2013 144 109
- HEATHER A.E. BENSON ET AL: "Enhanced skin permeation and hydration by magnetic field array: preliminary in-vitro and in-vivo assessment", JOURNAL OF PHARMACY AND PHARMACOLOGY, vol. 62, no. 6, 1 June 2010 (2010-06-01), pages 696-701, XP055425103, LONDON; GB ISSN: 0022-3573, DOI: 10.1211/jpp.62.06.0005

## Description

### TECHNICAL FIELD

A method and a device for the delivery of exogenous aggrecan precursors to the joint of a subject.

### BACKGROUND ART

The following discussion of the background art is intended to facilitate an understanding of the present disclosure only. The discussion is not an acknowledgement or admission that any of the material referred to is or was part of the common general knowledge as at the priority date of the application.

Joint pain and pathology is often caused by degeneration of the articular surfaces and cartilage in joints. Osteoarthritis (OA) affects more people than any other joint disease. It is the most prevalent form of arthritis and is a major source of disability in developed countries. Knee OA is the second most common form of OA and in the UK the pain associated with the condition is prevalent in 18-28% of people over 55. A prior history of joint injury is associated with an increased risk of OA while obesity is a major risk factor particularly for knee OA. A documented history of knee injury is also indicative of a greater risk (5 fold) in developing knee OA. Around 1.8% of the cartilage's absorbing power is lost every year from around 35 years of age. Even normal levels of sport and exercise can cause small but irreparable damage to articular surfaces resulting from bone-on-bone contact when the protective cartilage declines.

While there is no cure for the disease, primary treatment is aimed at pain reduction and improved joint mobility. Hence the cohort with significant prior knee injury is a key group to target for preventative interventions.

OA is characterized by loss and erosion of articular cartilage with osteophyte formation, changes in the synovial membrane, limited synovitis and changes in sub-chondral bone. Chondroitin sulfate and glucosamine sulfate are beneficial in their metabolic effect on various cell lines involved in the development of OA i.e. the chondrocytes, synovocytes and osteocytes from sub-chondral bone. These pro-aggrecans have been found to stimulate the increase in type II collagen and proteoglycan synthesis in human articular chondrocytes, reduce the activity of some of pro-inflammatory factors and reduce protease activity to improve the anabolic/catabolic balance of the extracellular cartilage matrix.

A range of medications are available. Current therapeutic regimens for treatment of degenerative joint disease mainly address pain, but do little to treat or prevent degeneration. Such therapies include analgesics such as acetaminophen (paracetamol) and non-steroidal anti-inflammatory drugs (NSAIDs). Injection of glucocorticoids (such as hydrocortisone) leads to short term pain relief that may last between a few weeks and a few months.

Oral chondroitin sulfate and glucosamine supplements are often administered for assistance in joint regeneration and supplementation of joint aggrecans. Oral steroids are not recommended in the treatment of degenerative joint disease because of their modest benefit and high rate of adverse effects. Joint injections of hyaluronic acid have not been found to lead to significant improvement and have been associated with significant harm.

Aggrecans (also known as cartilage-specific proteoglycan core proteins (CSPCP)) are a major structural component of cartilage, particularly articular cartilage, and are critical in the function of joints. The natural aggrecans found in healthy joints provides the ability for the tissues to withstand compressive loading and to maintain a near friction-less glide surface during articulation. The synthesis and degradation of aggrecans is associated with cartilage deterioration during joint injury, disease, and aging.

Aggrecans are large molecular weight proteoglycans, modified with chondroitin sulfate and glucosamine sulfate chains. The proteoglycans aggregate with glycosaminoglycans such as hyaluronan (also known as hyaluronic acid or HA), to form aggrecans. The properties of aggrecans are dependent on both the high charge density endowed by their numerous chondroitin and keratin sulfate chains; and their ability to form large molecular aggregates through interaction of the hyaluronan units. Glucosamine is a key component of keratin sulfate.

Such complex high molecular weight molecules are unsuitable for topical delivery due to their extremely poor transdermal penetration and lipophilicity that act to limit diffusion, and therefore bioavailability, to near zero. Dietary or oral supplementation of exogenous aggrecans has been unsuccessful due to aggressive first pass metabolism. It has been found that oral supplementation of exogenous aggrecans results in minimal changes in local aggrecan concentrations at the sight of joint injury or degeneration. Injectable compositions of aggrecans are not available, as regulatory approval for such complex mixtures of molecules are difficult to obtain.

US 2013/144109 relates to a method for the delivery of a skin care active agent comprising applying an active agent between a target dermal barrier and a magnetic device comprising one or more pairs of displaced dipolar magnetic elements linked by a magnetic return. Benson et al, 2010 (J. Pharm. Pharmacol.,62(6): 696-701) relates to a study aimed at determining the effect of magnetic film array technology on the skin permeation of urea.

There is therefore a need for methods to provide aggrecans and/or aggrecan precursors to joints to treat or prevent (or at least ameliorate) degenerative joint disease; or at least a method for complimenting the previously known treatment methods.

The present invention seeks to provide an improved or alternative delivery method for aggrecans that increases their delivery to joints.

It is against this background that the present invention has been developed.

### SUMMARY OF THE DISCLOSURE

The present invention is defined in the accompanying claims and relates to flexible magnetic films for use in treating joint pain or degeneration in a subject, devices for use in treatments or prevention of joint pain and degeneration in a subject, and kits for use in the delivery of exogenous aggrecan precursors to a joint of a subject that has joint pain or degeneration. Embodiments of the present invention are described in the following numbered paragraphs:
(1). A flexible magnetic film for use in treating joint pain or degeneration in a subject, wherein the film improves delivery of aggrecan precursors to a subject in need thereof, wherein the film is applied to the skin of the subject on a joint that has joint pain or degeneration, and wherein a composition comprising at least two aggrecan precursors chosen from the list consisting of hyaluronic acid, glucosamine sulfate and/or chondroitin sulfate is applied between the skin and the flexible magnetic film, and wherein at least part of the of the flexible magnetic film and composition are covered with a dressing, wherein the dressing increases the proprioception of the subject.
(2). The flexible magnetic film for use according to paragraph 1, wherein application of the film increases the penetration of the aggrecan precursors through the skin of the subject.
(3). The flexible magnetic film for use according to paragraph 2, wherein the increased penetration of the aggrecan precursors through the skin of the subject is by:
   (a) increased movement of the aggrecan precursors from the composition towards the skin of the subject; and/or
   (b) temporarily altering the permeability of the skin of the subject,
      optionally wherein the increased movement of the aggrecan precursors is via diamagnetic repulsion.
(4). A device for use in the treatment or prevention of joint pain and degeneration in a subject in need thereof, wherein the device comprises:
   a) a composition comprising at least two aggrecan precursors chosen from the list consisting of hyaluronic acid, glucosamine sulfate and/or chondroitin sulfate; and
   b) a flexible magnetic film; and,
   c) a dressing, wherein the dressing increases the proprioception of the subject, wherein the dressing covers at least part of the flexible magnetic film and composition;
      wherein the composition is applied between the skin of the subject and the flexible magnetic film, wherein the film is applied on a joint that has joint pain or degeneration.
(5). A kit for use in the delivery of exogenous aggrecan precursors to a joint of a subject that has joint pain or degeneration, wherein the kit comprises:
   a) a flexible magnetic film comprising at least one partially or completely self-adhesive surface;
   b) at least two aggrecan precursors chosen from the list comprising hyaluronic acid, glucosamine sulfate and/or chondroitin sulfate;
   c) a dressing with at least one self-adhesive surface; and,
   d) instructions for use.
(6). The kit according to paragraph 5, wherein the dressing increases the proprioception of the subject.
(7). The flexible magnetic film for use according to any one of paragraphs 1 to 3, device for use according to paragraph 4, or kit according to paragraph 5 or 6, wherein the composition comprises one of the following combinations: hyaluronic acid and glucosamine sulfate; hyaluronic acid and chondroitin sulfate; glucosamine sulfate and chondroitin sulfate; hyaluronic acid, glucosamine sulfate and chondroitin sulfate.
(8). The flexible magnetic film for use according to any one of paragraphs 1 to 3, device for use according to paragraph 4, or kit according to paragraph 5 or 6, wherein the subject is suffering from osteoarthritis.
(9). The flexible magnetic film for use according to any one of paragraphs 1 to 3, device for use according to paragraph 4, or kit according to paragraph 5 or 6, wherein the composition further comprises a sensate ingredient.
(10). The flexible magnetic film for use according to any one of paragraphs 1 to 3, device according to paragraph 4, or kit according to paragraph 5 or 6, wherein the aggrecan precursors are present in individual amounts of from about 0.001 to about 5% by weight of the composition.
(11). The flexible magnetic film for use according to any one of paragraphs 1 to 3, device for use according to paragraph 4, or kit according to paragraph 5 or 6, wherein the magnetic elements of the flexible magnetic film have the following parameters:
   i) a horizontal off-set of between about 1 and 10 millimetres, 2 and 8 millimetres, or 3 and 7 millimetres; and/or
   ii) a repetition rate of about 1 and 10 elements per centimetre, 1 and 6 elements per centimetre or 1.5 and 4 elements per centimetre; and/or
   iii) the poles of the magnetic elements in a particular spatial region are between about 1.0 mm to 10mm apart, or 1.0 mm to 5.0mm apart; and/or
   iv) the magnetic flux of each magnetic pole of the magnetic elements is between about 1mT and 100mT, 1mT to about 60mT, or 12mT to 45mT; and/or
   v) the delta flux between the magnetic flux of two adjacent poles of opposite polarity is between about 2mT and 200mT, 20mT to 140mT, or 20mT to 90mT.
(12). The flexible magnetic film for use, device for use, or kit according to paragraph 11 wherein the magnetic elements of the flexible magnetic film have a linear arrangement.
(13). The flexible magnetic film for use, device for use, or kit according to paragraph 12 wherein the magnetic elements of the flexible magnetic film have the following parameters: between 5 and 10 poles per cm and a magnetic flux of between 150 Gauss and 200 Gauss, optionally wherein the magnetic elements of the flexible magnetic film have the following parameters: 10 poles per cm and a magnetic flux of 172 Gauss.

The present disclosure provides a method for the delivery of exogenous aggrecan precursors to the joint of a subject, comprising the steps of:
a) applying a composition comprising one or more aggrecan precursors chosen from the list comprising hyaluronic acid, glucosamine sulfate and/or chondroitin sulfate to the skin of the subject on or near a joint, wherein the composition is applied between the skin and a flexible magnetic film.

The method preferably comprising the additional step of covering at least part of the composition and the flexible magnetic film with a dressing, wherein the dressing increases the proprioception of the subject.

Preferably, the composition comprises one of the following combinations: hyaluronic acid and glucosamine sulfate; hyaluronic acid and chondroitin sulfate; glucosamine sulfate and chondroitin sulfate; hyaluronic acid, glucosamine sulfate and chondroitin sulfate.

Preferably, the subject is suffering from joint pain or degeneration; more preferably osteoarthritis.

The present disclosure further provides a method for increasing the movement of aggrecan precursors from a composition towards the skin of a subject, comprising the step of:
a) applying a composition comprising one or more aggrecan precursors chosen from the list comprising hyaluronic acid, glucosamine sulfate and/or chondroitin sulfate to the skin of the subject on or near a joint, wherein the composition is applied between the skin and a flexible magnetic film.

The present disclosure further provides a method to temporarily alter the permeability of the skin of a subject to allow the penetration of aggrecan precursors, comprising the step of:
a) applying a composition comprising one or more aggrecan precursors chosen from the list comprising hyaluronic acid, glucosamine sulfate and/or chondroitin sulfate to the skin of the subject on or near a joint, wherein the composition is applied between the skin and a flexible magnetic film.

The present disclosure further provides a method for increasing the penetration of aggrecan precursors through the skin of a subject by (i) increasing the movement of the aggrecan precursors from a composition towards the skin; and (ii) temporarily altering the permeability of the skin to allow the penetration of the aggrecan precursors; comprising the step of:
a) applying a composition comprising one or more aggrecan precursors chosen from the list comprising hyaluronic acid, glucosamine sulfate and/or chondroitin sulfate to the skin of the subject on or near a joint, wherein the composition is applied between the skin and a flexible magnetic film.

The present disclosure further provides a method for the delivery of aggrecan precursors to the joint of a subject, comprising the steps of:
a) applying a composition comprising one or more of hyaluronic acid, glucosamine sulfate and/or chondroitin sulfate to the skin of the subject on or near a joint, wherein the composition is applied between the skin and a flexible magnetic film; and
b) covering at least part of the composition comprising aggrecan precursors and flexible magnetic film with a dressing, wherein the dressing increases the proprioception of the subject.

The present disclosure further provides a device for the treatment or prevention of joint pain and degeneration, comprising:
a) a composition comprising one or more aggrecan precursors chosen from the list comprising hyaluronic acid, glucosamine sulfate and/or chondroitin sulfate; and
b) a flexible magnetic film
wherein the composition is applied between the skin and the flexible magnetic film.

The device above, further comprising a dressing, wherein the dressing increases the proprioception of the subject and wherein the dressing covers at least part of the composition and flexible magnetic film.

The disclosure further provides a kit for the delivery of exogenous aggrecan precursors to the joint of a subject comprising:
a) a flexible magnetic film comprising at least one partially or completely self-adhesive surface;
b) aggrecan precursors chosen from the list comprising hyaluronic acid, glucosamine sulfate and/or chondroitin sulfate;
c) instructions for use.

The disclosure further provides a kit for the delivery of exogenous aggrecan precursors to the joint of a subject comprising:
a) a flexible magnetic film;
b) aggrecan precursors chosen from the list comprising hyaluronic acid, glucosamine sulfate and/or chondroitin sulfate;
c) a dressing with at least one self-adhesive surface;
d) instructions for use.

### BRIEF DESCRIPTION OF THE DRAWINGS

The description will be made with reference to the accompanying drawings in which:
Figure 1 is a diagrammatic representation of an example flexible magnetic film comprising a complex magnetic field according to the present disclosure.
Figure 2 is a schematic of a patch that may be used to assist in the delivery of the compositions of the present disclosure to a joint.
Figure 3 is a graph of the % Improvement for timed performance tests after delivery of the composition according to the present disclosure to the skin of subjects.
Figure 4 is a graph of the % Improvement for timed measured distance tests after delivery of the composition according to the present disclosure to the skin of subjects.
Figure 5 is a graph of the % Improvement for timed measured distance tests after delivery of the composition according to the present disclosure to the skin of subjects.
Figure 6 is a graph of the Penetration of glucosamine through excised human epidermis as determined from Franz cell diffusion studies.
Figure 7 is a graph of the Loading of Glucosamine (ug) in the Stratum Corneum determined by tape stripping.

### DESCRIPTION OF THE DISCLOSURE

### Detailed Description of the Disclosure

It is known that one of the contributing factors to joint degradation and pain is the depletion of aggrecans in the cartilage of the joints. Currently, attempts to rectify this issue have involved injection of hyaluronan or oral supplementation of glucosamine or chondroitin. However, to date there has been no therapeutically effective and regulatorally acceptable method of directly supplementing the aggrecan population in joints.

The present inventors have unexpectedly found that compositions compromising a combination of aggrecan precursors can be delivered through the skin to the joint for the treatment or prevention of degenerative joint disease using a magnetic microarray. The present disclosure combines diamagnetic forces from an integrated magnetic microarray to deliver therapeutic actives through the skin at the site of the affected joint, thus bypassing the first pass metabolic fate of orally ingested actives. The active ingredients include glucosamine sulfate, chondroitin sulfate and/or hyaluronic acid, which, due to their relatively large molecular weights, are difficult to induce to penetrate the outer layers of skin. With present combination of diamagnetic technology and a composition comprising aggrecan precursors, there is provided enhanced bioavailability of these key pro-aggrecans in the tissue surrounding the affected joint.

The present disclosure provides a method for the delivery of exogenous aggrecan precursors to the joint of a subject, comprising the steps of:
a) applying a composition comprising one or more aggrecan precursors chosen from the list comprising: hyaluronic acid, glucosamine sulfate and/or chondroitin sulfate to the skin of the subject on or near a joint, wherein the composition is applied between the skin and a flexible magnetic film.

The present disclosure further provides a method for the delivery of exogenous aggrecan precursors to the joint of a subject, comprising the steps of:
a) applying a composition comprising the aggrecan precursors hyaluronic acid and glucosamine sulfate to the skin of the subject on or near a joint, wherein the composition is applied between the skin and a flexible magnetic film.

The present disclosure further provides a method for the delivery of exogenous aggrecan precursors to the joint of a subject, comprising the steps of:
a) applying a composition comprising the aggrecan precursors glucosamine sulfate and chondroitin sulfate to the skin of the subject on or near a joint, wherein the composition is applied between the skin and a flexible magnetic film.

The present disclosure further provides a method for the delivery of exogenous aggrecan precursors to the joint of a subject, comprising the steps of:
a) applying a composition comprising the aggrecan precursors hyaluronic acid and chondroitin sulfate to the skin of the subject on or near a joint, wherein the composition is applied between the skin and a flexible magnetic film.

The present disclosure further provides a method for the delivery of exogenous aggrecan precursors to the joint of a subject, comprising the steps of:
a) applying a composition comprising the aggrecan precursors hyaluronic acid, glucosamine sulfate and chondroitin sulfate to the skin of the subject between the skin and a flexible magnetic film.

Preferably, the present disclosure assists in the avoidance of joint degeneration (or prevention of further joint degeneration if damage has already occurred), improves movement and exercise performance and reduces exercise recovery times. For example, the method may be used in the treatment or prevention of joint degeneration and pain (such as that associated with osteoarthritis), in joints such as fingers, hands, wrists, elbows, shoulders, necks, the spine, hips, knees, feet and/or toes. The disclosure preferably slows the breakdown of cartilage in the joint. The treatment or prevention of joint degeneration and reduced breakdown of cartilage may preferably be accompanied by a reduction in pain.

The present disclosure further provides a method for the delivery of exogenous aggrecan precursors to the joint of a subject, comprising the steps of:
a) applying a composition comprising one or more aggrecan precursors chosen from the list comprising: hyaluronic acid, glucosamine sulfate and/or chondroitin sulfate to the skin of the subject on or near a joint, wherein the composition is applied between the skin and a flexible magnetic film; and
b) covering at least part of the composition and the flexible magnetic film with a dressing, wherein the dressing increases the proprioception of the subject.

The flexible magnetic film generates a magnetic field which serves to (a) increase the partitioning of the exogenous aggrecan precursors from the composition in which they are held, allowing more of the aggrecan precursors to be able to move through the skin; and/or (b) temporarily modify or alter the barrier function and permeability of the skin, changing the microfluidic flow of the skin without permanently changing the physical structure of the surface, and allowing the penetration of the aggrecan precursors.

Most pharmaceutical compositions (such as emulsions) are designed to be stable for an extended shelf life. However, this very stability means that the aggrecan precursors generally have no impetus to come out of the composition in which they held, and thus less of the active is free to move into the skin surface. The flexible magnetic film of the present disclosure overcomes the natural reluctance of the aggrecan precursors to leave the stable composition they are provided in, and increases the movement of the aggrecan precursors from the composition, thus releasing more of the aggrecan precursors to penetrate the skin. Increasing the magnetic flux within certain limits increases the diamagnetic repulsion of the aggrecan precursors away from the magnetic source and towards the skin barrier. This is a feature of diamagnetic susceptibility and is related to the paired electrons of diamagnetic molecules being repelled by magnetic fields. In this way, diamagnetic repulsion provides a means of adding directionality and mobility to molecules during diffusion. However, increasing the magnetic flux to very high levels, or the use of strong magnets such as those with a magnetic flux density of 5,000-50,000 Gauss or more, will not work in providing suitable diamagnetic repulsion, as they increase the entropic motion of the components of the composition, and this increase in the randomness of the motion of the elements results in the components not moving in the specific directions desired.

The method of enhancing delivery of aggrecan precursors through the skin involves the utilization of magnetic principles to apply force upon the aggrecan precursors in such a manner as to ensure that the force acting on the active agents is different from that acting on the molecules of the composition the aggrecan precursors are provided in. As a result, another method of improving the utility of the disclosure is to select or chemically alter the diamagnetic sensitivity of the aggrecan precursors or of the composition the aggrecan precursors are provided in, with the view to enhancing the differences in diamagnetic sensitivity between the two entities. By way of example, the additional of a light ester such as phenxyethyl acrylate to a diethylaminoethyl acrylate polymer may act to increase the diamagnetic susceptibility of the polymer and by doing so increase the delivery of the aggrecan precursors from the composition in which they are provided.

The present disclosure also provides a method for increasing the movement of aggrecan precursors from a composition towards the skin of a subject, comprising the step of:
a) applying a composition comprising one or more aggrecan precursors chosen from the list comprising hyaluronic acid, glucosamine sulfate and/or chondroitin sulfate to the skin of the subject on or near a joint, wherein the composition is applied between the skin and a flexible magnetic film.

The disclosure also provides a method to temporarily alter the permeability of the skin of a subject to allow the penetration of aggrecan precursors , comprising the step of
a) applying a composition comprising one or more aggrecan precursors chosen from the list comprising hyaluronic acid, glucosamine sulfate and/or chondroitin sulfate to the skin of the subject on or near a joint, wherein the composition is applied between the skin and a flexible magnetic film.

The present disclosure further provides a method for increasing the penetration of aggrecan precursors through the skin of a subject by (i) increasing the movement of the aggrecan precursors from a composition towards the skin; and (ii) temporarily altering the permeability of the skin to allow the penetration of the aggrecan precursors; comprising the step of:
a) applying a composition comprising one or more aggrecan precursors chosen from the list comprising hyaluronic acid, glucosamine sulfate and/or chondroitin sulfate to the skin of the subject on or near a joint, wherein the composition is applied between the skin and a flexible magnetic film.

The composition may further comprise sensate ingredients to increase the subject's in-use skin feel characteristics. Preferably, the sensate ingredients are chosen from the list comprising: menthol, thymol, menthyl lactate, camphor, eucalyptus oil, essential oils, n-N-substituted-p-menthane-3-carboxamides, acyclic tertiary and secondary carboxamides, 3-1-menthoxy propan-1,2-diol, vanillyl alcohol n-butyl ether, vanillyl alcohol n-propyl ether, vanillyl alcohol isopropyl ether, vanillyl alcohol isobutyl ether, vanillyl alcolol n-amino ether, vanillyl alcohol isoamyl ether, vanillyl alcohol n-hexyl ether vanillyl alcohol methyl ether, vanillyl alcohol ethyl ether, gingerol, shogaol, paradol, zingerone, capsaicin, dihydrocapsaicin, nordihydrocapsaicin, homocapsaicin, homodihydrocapsaicin, ethanol, benzyl alcohol, chloroform, eugenol, cinnamon oil, cinnamic aldehyde. Sensate ingredients were included in the composition to increase the proprioceptive response from the subject when the composition is applied to the skin.

The composition comprising aggrecan precursors delivered by the method of the present disclosure is preferably in the form of a gel, paste or liquid.

The flexible magnetic film of the present disclosure preferably comprises magnetic elements, particles, fragments, or flakes disposed in a flexible solid or semi-solid matrix. The flexible magnetic films may be used in any of the methods or devices discussed above.

In the flexible magnetic film, each individual magnetic element provides diamagnetic repulsion to the aggrecan precursors which are to be transported across the skin barrier, whilst the horizontal flux between the various magnetic elements acts to polarize the dielectric properties of the target skin tissue, inducing permeation changes.

Generally, the magnetic elements of the flexible magnetic film have a horizontal offset between centres of between 1 and 10 millimetres, preferably between 2 and 8 millimetres, more preferably between 3 and 7 millimetres. The horizontal offsets may be 1mm, 2mm, 3mm, 4mm, 5mm, 6mm, 7mm, 8mm, 9mm or 10mm, or any range between.

As a result, the magnetic elements may be disposed at a repetition rate of between 1 and 10 elements per centimetre, preferably between 1 and 6 elements per centimetre more preferably 1.5 and 4 elements per centimetre. The magnetic elements may be disposed at a repetition rate of 1 elements per centimetre, 1.5 elements per centimetre, 2 elements per centimetre, 2.5 elements per centimetre, 3 elements per centimetre, 3.5 elements per centimetre, 4 elements per centimetre, 4.5 elements per centimetre, 5 elements per centimetre, 5.5 elements per centimetre, 6 elements per centimetre, 6.5 elements per centimetre, 7 elements per centimetre, 7.5 elements per centimetre, 8 elements per centimetre, 8.5 elements per centimetre, 9 elements per centimetre, 9.5 elements per centimetre, or 10 elements per centimetre, or any range between.

Preferably, the poles of the magnetic elements in a particular spatial region are between 1.0 mm to 10mm apart, more preferably the poles are between 1.0 mm to 5.0mm apart. For example, the poles may be 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0 mm apart, or any range between.

In another aspect of the disclosure, the magnetic flux of each magnetic pole of the magnetic elements is between about 1mT and about 100mT. Preferably, the flux of each pole is between about 1mT to about 60mT, most preferably about 12mT to 45mT. For example, the magnetic flux may be 1mT, 5mT, 10mT, 11mT, 12mT, 13mT, 14mT, 15mT, 20mT, 25mT, 30mT, 35mT, 40mT, 45mT, 50mT, 55mT, 60mT, 65mT, 70mT, 75mT, 80mT, 85mT, 90mT, 9 5mT or 100mT, or any range between.

In another aspect, the difference or delta flux between the magnetic flux of two adjacent poles of opposite polarity is between about 2mT and about 200mT. More preferably, the difference between the magnetic flux of two adjacent poles of opposite polarity is between about 20mT to about 140mT, most preferably about 20mT to 90mT. The delta flux may therefore be 2mT, 5mT, 10mT, 20mT, 25mT, 30mT, 35mT, 40mT, 45mT, 50mT, 55mT, 60mT, 65mT, 70mT, 75mT, 80mT, 85mT, 90mT, 9 5mT, 100mT, 105mT, 110mT, 115mT, 120mT, 130mT, 140mT, 150mT, 160mT, 170mT, 180mT, 190mT, 200mT, or any range between.

Preferably, the magnetic elements of the flexible magnetic film have the following parameters:
i) a horizontal off-set of between about 1 and 10 millimetres, 2 and 8 millimetres, or 3 and 7 millimetres; and/or
ii) a repetition rate of about 1 and 10 elements per centimetre, 1 and 6 elements per centimetre or 1.5 and 4 elements per centimetre; and/or
iii) the poles of the magnetic elements in a particular spatial region are between about 1.0 mm to 10mm apart, or 1.0 mm to 5.0mm apart; and/or
iv) the magnetic flux of each magnetic pole of the magnetic elements is between about 1mT and 100mT, 1mT to about 60mT, or 12mT to 45mT; and/or
v) the delta flux between the magnetic flux of two adjacent poles of opposite polarity is between about 2mT and 200mT, 20mT to 140mT, or 20mT to 90mT.

The magnetic elements in the flexible magnetic film may be arranged in a linear fashion, with rows of N and S elements forming a simple linear pattern.

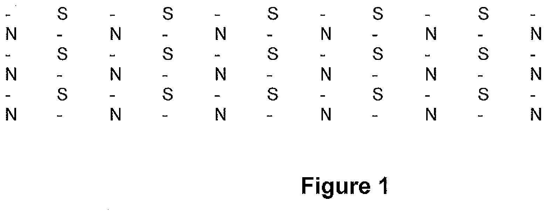

The magnetic elements in the flexible magnetic film may alternatively generate a complex field that has three separate polarities - neutral, north and south. Figure 1 is a diagrammatic representation of an example diamond arrangement field, wherein neutral [shown as -] has magnetic flux of between 0 - 30mT, north [shown as N] has magnetic flux of between 25mT and 50mT and south [shown as S] has magnetic flux of between -25mT and -50mT. Arrangement of the three polarities creates a series of S, N, S, N diamond shapes, with a neutral flux in the centre, linked together by proximity with the adjoining diamond shape.

For example, across line one of Figure 1, the fluxes might be 20mT, -45mT, 20mT, - 45mT; across line two it might be 45mT, 20mT, 45mT, 20mT.

The strongest magnetic gradient would be located between the north and south poles. Preferably the gradient is between 100mT and 50mT, more preferably 90mT.

To create such a complex field on a flexible magnetic film, one may start with a standard linear parallel flux pattern of N, S, N, S wherein each flux is, for example, 45mT (ie the N are all 45mT, and the S are all -45mT). This may then be crossed or angularly offset at 90 degrees with another N, S, N, S flux pattern of, for example, 25mT (ie the N are all 25mT, and the S are all -25mT). Where the N and S of the two flux patterns overlap, the magnetic fluxes cancel each other out, resulting in a neutral flux of, for example, 20mT (eg a 45mT N cancels out a -25mT S to leave a neutral 20mT flux). Such complex fields are preferably generated by overlaying two linear arrays at an angular offset of between 1 degree and 90 degrees, for example 5, 10, 15, 20, 25, 30, 35, 40, 45, 50, 55, 60, 65, 70, 75, 80, 85 or 90 degree angular offset.

Preferably the flexible magnetic film is between 0.5 and 1.2mm thickness.

Preferably, the magnetic elements or particles of the flexible magnetic film are disposed in a solid or semi-solid matrix or base. The solid or semi-sold matrix may comprise a flexible matrix chosen from the list comprising, but not limited to: rubber, silicone, polyethylene terephthalate (PET), polyethylene naphthalate (PEN), polyethersulphone (PES), polyimide (PI), polydimethylsiloxane (PDMS), grapheme, poly (methyl methacrylate) (PMMA). The magnetic film may be produced by combining pre-magnetised elements with the solid or semi-solid matrix, or non-magnetised elements may be provided in a solid or semi-solid matrix and the required magnetic pattern is then impressed upon the elements.

The flexible magnetic film of the present disclosure may be constructed using a range of magnetic materials exhibiting ferromagnetic properties. Preferably, the material is a ferromagnetic material such as an iron compound (e.g. a ferrite such as barium ferrite, magnetite, or mild steel), a cobalt material, a strontium material, a barium material or a nickel material; optionally with a metalloid component such as boron, carbon, silicon, phosphorus or aluminium. Alternately, rare-earth materials such as neodymium or samarium-cobalt may also be used.

The magnetic array may be held in place on or near the joint using tape such as self-adhesive tape, a bandage, a patch (preferably a self-adhesive patch) or other securing means.

While the aggrecan precursors may be provided and used alone with the flexible magnetic film, in many situations the aggrecan precursors will be included in a composition either alone or in combination with one or more other active agents.

The composition employed in the present disclosure to deliver the aggrecan precursors may include additives such as other buffers, diluents, carriers, adjuvants or excipients. Any pharmacologically acceptable buffer that is magnetically inert or neutral; or which has a magnetic susceptibility that is either paramagnetic in nature or is more paramagnetic than the aggrecan precursors being delivered, may be used. For example tris buffers or phosphate buffers.

Other agents may be employed in the composition comprising aggrecan precursors for a variety of purposes. For example, buffering agents, preservatives, co-solvents, surfactants, oils, humectants, emollients, chelating agents, stabilizers or antioxidants may be employed. Preservatives which may be employed include, but are not limited to: benzalkonium chloride, chlorobutanol, thimerosal, sodium bisulfate, phenylmercuric acetate, phenylmercuric nitrate, ethyl alcohol, methylparaben, polyvinyl alcohol, benzyl alcohol, germaben, parabens, imidureas, kathon and phenylethyl alcohol. The composition may further comprise a surfactant such as Tween 80. Other components of the composition may include, but are not limited to, polyvinyl alcohol, povidone, hydroxypropyl methyl cellulose, poloxamers, carboxymethyl cellulose, hydroxyethyl cellulose, purified water, etc. Tonicity adjustors and electrolytes such as, for example, sodium chloride, potassium chloride, mannitol, glycerin, etc may also be included. Antioxidants include, but are not limited to, sodium metabisulfite, sodium thiosulfate, acetylcysteine, butylated hydroxyanisole, butylated hydroxytoluene, etc.

Suitable buffering agents that may be employed in the composition comprising the aggrecan precursors may include sodium carbonate, sodium borate, sodium phosphate, sodium acetate, sodium bicarbonate, etc., as approved by the US FDA for the desired route of administration. These agents may be present in amounts sufficient to maintain a pH of the system of between about 2 to about 9, preferably about 4 to about 8, more preferably 4.5, 5, 5.5, 6, 6.5, 7 or 7.5 (or any pH in between). As such the buffering agent may be as much as about 5% on a weight to weight basis of the total composition.

The composition preferably comprises, along with the aggrecan precursors, solvents such as propylene glycol, water and ethanol; preservatives such as germaben; viscosity modifying agents such as gelatine; vitamins such as vitamin C and E. The composition may further comprise agents such as colouring agents and/or perfuming agents such as essential oils, esters etc.

The indications, effective doses, contra-indications, vendors etc, of aggrecan precursors are available or are known to one skilled in the art.

The aggrecan precursors may be present in individual amounts of from about 0.001 to about 5% by weight of the composition and preferably about 0.01% to about 2% by weight of the composition. However, it is contemplated that the aggrecan precursors may be present in individual amounts greater than this, for example up to 100% by weight of the composition.

Preferably, the glucosamine sulfate is provided at a concentration of between about 0.1% by weight of the composition and 10% by weight of the composition, more preferably between about 0.05% by weight of the composition and about 5% by weight of the composition. For example, the glucosamine sulfate may be provided at about 0.1%, 0.2%, 0.25%, 0.5%, 0.6%, 0.7%, 0.75%, 0.8%, 0.9%, 1%, 1.25%, 1.5%, 2%, 3%, 4%, 5%, 6%, 7%, 8% 9% or 10% by weight of the composition. Most preferably the glucosamine sulfate is provided at a concentration of between about 0.5% and 1.5%, or about 1% by weight of the composition.

Preferably, the hyaluronic acid is provided at a concentration of between about 0.01% by weight of the composition and 10% by weight of the composition, more preferably between about 0.05% by weight of the composition and about 5% by weight of the composition. For example, the glucosamine sulfate may be provided at about 0.01%, 0.02%, 0.05%, 0.075%, 0.08%, 0.09%, 0.1%, 0.2%, 0.25%, 0.3%, 0.4%, 0.5%, 0.6%, 0.7%, 0.75%, 0.8%, 0.9%, 1%, 1.25%, 1.5%, 2%, 3%, 4%, 5%, 6%, 7%, 8% 9% or 10% by weight of the composition. Most preferably the hyaluronic acid is provided at a concentration of between about 0.1% and 0.6%, or about 0.25% by weight of the composition.

Preferably, the chondroitin sulfate is provided at a concentration of between about 0.01% by weight of the composition and 10% by weight of the composition, more preferably between about 0.05% by weight of the composition and about 5% by weight of the composition. For example, the glucosamine sulfate may be provided at about 0.01%, 0.02%, 0.05%, 0.075%, 0.08%, 0.09%, 0.1%, 0.2%, 0.25%, 0.3%, 0.4%, 0.5%, 0.6%, 0.7%, 0.75%, 0.8%, 0.9%, 1%, 1.25%, 1.5%, 2%, 3%, 4%, 5%, 6%, 7%, 8% 9% or 10% by weight of the composition. Most preferably the chondroitin sulfate is provided at a concentration of between about 0.1% and 0.6%, or about 0.25% by weight of the composition.

The aggrecan precursors may be provided in a matrix. If the aggrecan precursors are contained within a matrix, the matrix preferably allows the aggrecan precursors to diffuse or exit the matrix in some manner and contact the skin.

The matrix is preferentially prepared from a polymer or copolymer prepared from e.g., polyisobutylene, ester of polyvinyl alcohol, polyacrylic and polymethacrylic acid esters, natural rubber, polymers of styrene, isoprene, and styrene-butadiene or silicone polymers, resin components, such as, saturated and unsaturated hydrocarbon resins, derivatives of abietyl alcohol and of beta-pinene, plasticizers, such as phthalic acid esters, triglycerides and fatty acids, as well as a series of other substances known to those skilled in the art.

Matrix biocompatible polymers that might be used in the disclosure include compounds such as polycaprolactone, polyglycolic acid, polylactic acid, polyanhydrides, polylactide-co-glycolides, polyamino acids, polyethylene oxide, acrylic terminated polyethylene oxide, polyamides, polyethylenes, polyacrylonitriles, polyphosphazenes, poly(ortho esters), sucrose acetate isobutyrate (SAIB), and other polymers such as those disclosed in U.S. Patent Nos. 6,667,371; 6,613,355; 6,596,296; 6,413,536; 5,968,543; 4,079,038; 4,093,709; 4,131,648; 4,138,344; 4,180,646; 4,304,767; 4,946,931.

The matrix containing the aggrecan precursors may also be prepared from thermosetting polymers such as tetra-substituted ethylene diamine block copolymers of ethylene oxide and propylene oxide (e.g., poloxamine); polycarbophil; and polysaccharides such as gellan, carrageenan (e.g., kappa-carrageenan and iota-carrageenan), chitosan and alginate gums.

The matrix may also be a hydrogel, being a gel prepared with hydrophilic polymers, and these materials are well known in the art. Examples of hydrophilic polymers useful for the preparation of hydrogels are polyacrylate, polymethacrylate, polyacrylamide, poly(vinyl alcohol), poly(ethylene oxide), poly(ethylene imine), carboxy-methylcellulose, methylcellulose, poly(acrylamide sulphonic acid), polyacrylonitrile, poly(vinyl-pyrrolidone), agar, dextran, dextrin, carrageenan, xanthan, and guar. The preferred hydrogels are acrylates and may be, for example, preferably made from acrylic esters of quaternary chlorides and/or sulfates or acrylic amides of quaternary chlorides; polymers of this type are disclosed in U.S. Pat. No. 5,800,685. The hydrophilic polymers will generally constitute from about 1 to about 70%, preferably about 5 to about 60%, more preferably about 10 to about 50%, by weight of the hydrogel.

The composition comprising aggrecan precursors may optionally be retained in a scaffold, such as foam, silicon gel, wadding (such as cotton wadding) etc. Alternatively or in addition, the composition comprising aggrecan precursors may be held in a reservoir or flexible pouch, with one surface of the reservoir or pouch being constructed such that the aggrecan precursors can penetrate the surface and exit the reservoir or pouch (for example, the surface may be perforated or made of a material that is permeable to at least the aggrecan precursors). This surface of the reservoir or pouch would be applied to the skin surface of the subject to whom the aggrecan precursors are being delivered.

The optional scaffold and/or reservoir or pouch may be held against the skin by the flexible magnetic film, preferably wherein the flexible magnetic film comprises at least one partially or completely self-adhesive surface. Alternatively, both the optional scaffold and/or reservoir or pouch and the flexible magnetic film may be held against the skin by a dressing or bandage, preferably a dressing or bandage with at least one self-adhesive surface. The optional scaffold and/or reservoir or pouch may be permanently attached to the flexible magnetic film or the dressing; or may be removable, such that the flexible magnetic film or the dressing may be re-used by insertion of a fresh scaffold and/or reservoir or pouch between the flexible magnetic film or dressing and the skin.

The composition comprising aggrecan precursors can also be simply applied to the skin of the subject to whom the aggrecan precursors are being delivered by rubbing or smearing the composition onto the skin, prior to application of the flexible magnetic film. The flexible magnetic film may cover the applied composition comprising aggrecan precursors and hold it in the preferred location, preferably wherein the flexible magnetic film comprises at least one partially or completely self-adhesive surface; or the applied composition comprising aggrecan precursors and the flexible magnetic film may be held in place on the skin with a dressing or bandage, preferably a dressing or bandage with at least one self-adhesive surface.

The self-adhesive surface of the flexible magnetic film or dressing may be covered by a protective liner, which is removed before application of the flexible magnetic film or dressing to the skin. The protective liner may further cover the optional scaffold and/or reservoir or pouch (if present), and assist in retaining the composition comprising aggrecan precursors in the scaffold or reservoir until required.

Without wishing to be held to a theory, we believe that the high charge density endowed on aggrecans by the numerous chondroitin and keratin sulfate chains, that provide the aggrecans with their ability to form large molecular aggregates with hyaluronans, do not form automatically or in response to simple proximity.

We have discovered that the *in situ* and *in vivo* aggregation of the numerous chondroitin and keratin sulfate chains with hyaluronans requires oscillation of the charge densities. This oscillation is generated by the deformation of the aggrecan proteoglycans in the cartilage matrix in response to physical loading. The property of growth regulation through mechanical loading of osseous tissues is well known. However the discovery of the same bio-response to exercise by cartilage proteoglycans, and its role in regulating the turnover of aggrecans and the rate of aggrecans formations, is a new discovery. Therefore, the method of the present disclosure, wherein exogenous aggrecan precursors such as hyaluronic acid, glucosamine sulfate and/or chondroitin sulfate (a key element of keratin sulfate) are delivered through the skin into the joint, may be enhanced by increasing the subject's proprioception and exercise levels once the active agents have been delivered.

In order to harness this effect, the present disclosure provides a method for the delivery of aggrecan precursors to the joint of a subject, comprising the steps of:
a) applying a composition comprising one or more of hyaluronic acid, glucosamine sulfate and/or chondroitin sulfate to the skin of the subject on or near a joint, wherein the composition is applied between the skin and a flexible magnetic film; and
b) covering at least part of the composition comprising aggrecan precursors and flexible magnetic film with a dressing, wherein the dressing increases the proprioception of the subject.

The presence of the dressing increases the subject's awareness of the movement of the joint being treated by the application of the composition comprising aggrecan precursors. The presence of the dressing also provides additional support to the subject's joint and muscles and increases their propensity and willingness to exercise. The increases exercise in turn increases the ability of the aggrecan precursors to form aggrecans. Thus the presence of the dressing enhances and synergistically increases the effectiveness of the method.

The dressing of the method of the present disclosure preferably allows movement and exercise during application. The subject preferably conducts exercise, preferably exercises of the joint to which the composition comprising aggrecan precursors has been delivered, whilst applying the method. The movement increases the ability of the exogenous and endogenous aggrecan precursors to combine and form aggrecans within the joint, by oscillation of the charge densities of the numerous chondroitin and keratin sulfate chains. This oscillation though joint movement serves to increase the association of the aggrecan precursors (ie the association of the keratin sulfate and chondroitin sulfate chians with the hyaluronic acid core) to form aggrecans. Thus, the composition provides the aggrecan precursors (hyaluronic acid, glucosamine sulfate and/or chondroitin sulfate), the flexible magnetic film delivers the aggrecan precursors through the skin into the joint, and the dressing increases the subject's ability to exercise and thus form the aggrecan precursors into aggrecans.

The term "dressing" encompasses any bandages, tape, plasters and dressings which provide proprioceptive feedback to a wearer. The dressing may also serve to hold the flexible magnetic film and/or composition comprising aggrecan precursors against the skin of a subject in a desired location. Preferably, the dressing has at least one partially or wholly self-adhesive surface. This adhesive surface both increases the wearer's awareness of the dressing and therefore the proprioceptive feedback; and also holds the composition and flexible magnetic film in place on the subject's skin, such that the optimal delivery of active agents through the skin to the joint is maintained.

Preferably the dressing comprises a pressure sensitive tape, plaster or bandage, with a hypoallergenic adhesive which is designed to hold firmly onto skin, dressing materials, and underlying layers of tape. For example, the dressing may be kinesiology tape or bandage, or 3M Micropore® tape. The dressing is preferably porous and/or breathable, and may be made of cotton. Preferably the dressing is water proof and may be worn for several days.

Preferably the dressing is a kinesiology bandage. Preferably the kinesiology bandage is very thin and made with a porous cotton fabric. The fabric lets the skin breathe and has an elasticity comparable to that of the skin and muscles. The sticky side of the tape may use a water-repellent, medical-grade acrylic adhesive that further supports the muscles and connective tissues.

The present disclosure provides a device for the treatment or prevention of joint pain and degeneration, comprising
a) a composition comprising one or more aggrecan precursors chosen from the list comprising hyaluronic acid, glucosamine sulfate and/or chondroitin sulfate; and
b) a flexible magnetic film
   wherein the composition is applied between the skin and the flexible magnetic film.

The present disclosure further provides a device for the treatment or prevention of joint pain and degeneration, comprising
a) a composition comprising one or more aggrecan precursors chosen from the list comprising hyaluronic acid, glucosamine sulfate and/or chondroitin sulfate;
b) a flexible magnetic film; and
c) a dressing, wherein the dressing increases the proprioception of the subject.
   wherein the composition is applied between the skin and the flexible magnetic film and the dressing covers at least part of the composition and flexible magnetic film.

Preferably the composition comprises one of the following combinations: hyaluronic acid and glucosamine sulfate; hyaluronic acid and chondroitin sulfate; glucosamine sulfate and chondroitin sulfate; hyaluronic acid, glucosamine sulfate and chondroitin sulfate.

Preferably the joint pain and degeneration is osteoarthritis.

The flexible magnetic film is as described above. Preferably, the magnetic elements of the flexible magnetic film have the following parameters:
vi) a horizontal off-set of between about 1 and 10 millimetres, 2 and 8 millimetres, or 3 and 7 millimetres; and/or
i) a repetition rate of about 1 and 10 elements per centimetre, 1 and 6 elements per centimetre or 1.5 and 4 elements per centimetre; and/or
ii) the poles of the magnetic elements in a particular spatial region are between about 1.0 mm to 10mm apart, or 1.0 mm to 5.0mm apart; and/or
iii) the magnetic flux of each magnetic pole of the magnetic elements is between about 1mT and 100mT, 1mT to about 60mT, or 12mT to 45mT; and/or
iv) the delta flux between the magnetic flux of two adjacent poles of opposite polarity is between about 2mT and 200mT, 20mT to 140mT, or 20mT to 90mT.

The magnetic elements in the flexible magnetic film may be arranged in a linear fashion, with rows of N and S elements forming a simple linear pattern. The magnetic elements in the flexible magnetic film may alternatively be arranged to generate a complex diamond magnetic field.

The term "dressing" encompasses any bandages, tape, plasters and dressings which provide proprioceptive feedback to a wearer. The dressing may also serve to hold the flexible magnetic film and/or composition comprising aggrecan precursors against the skin of a subject in a desired location. Preferably, the dressing has at least one partially or wholly self-adhesive surface. This adhesive surface both increases the wearer's awareness of the dressing and therefore the proprioceptive feedback; and also holds the composition and flexible magnetic film in place on the subject's skin, such that the optimal delivery of active agents through the skin to the joint is maintained.

Preferably the dressing comprises a pressure sensitive tape, plaster or bandage, with a hypoallergenic adhesive which is designed to hold firmly onto skin, dressing materials, and underlying layers of tape. For example, the dressing may be kinesiology tape or bandage, or 3M Micropore® tape. The dressing is preferably porous and/or breathable, and may be made of cotton. Preferably the dressing is water proof and may be worn for several days.

Preferably the dressing is a kinesiology bandage. Preferably the kinesiology bandage is very thin and made with a porous cotton fabric. The fabric lets the skin breathe and has an elasticity comparable to that of the skin and muscles. The sticky side of the tape may use a water-repellent, medical-grade acrylic adhesive that further supports the muscles and connective tissues.

The device of the present disclosure preferably allows movement and exercise during application. The subject preferably conducts exercise, preferably exercises of the joint to which the composition comprising aggrecan precursors has been delivered, whilst wearing the device. The movement increases the ability of the exogenous and endogenous aggrecan precursors to combine and form aggrecans within the joint, by oscillation of the charge densities of the numerous chondroitin and keratin sulfate chains. This oscillation though joint movement serves to increase the association of the aggrecan precursors (ie the association of the keratin sulfate and chondroitin sulfate chians with the hyaluronic acid core) to form aggrecans. Thus, the composition provides the aggrecan precursors (hyaluronic acid, glucosamine sulfate and/or chondroitin sulfate), the flexible magnetic film delivers the aggrecan precursors through the skin into the joint, and the dressing increases the subject's ability to exercise and thus form the aggrecan precursors into aggrecans.

The aggrecans in the device may be held in a reservoir or flexible pouch and/or in a scaffold.

The present disclosure further provides a kit comprising:
a) a flexible magnetic film comprising at least one partially or completely self-adhesive surface;
b) aggrecan precursors chosen from the list comprising hyaluronic acid, glucosamine sulfate and/or chondroitin sulfate;
c) instructions for use.

The present disclosure further provides a kit comprising:
a) a flexible magnetic film;
b) aggrecan precursors chosen from the list comprising hyaluronic acid, glucosamine sulfate and/or chondroitin sulfate;
c) a dressing with at least one self-adhesive surface;
d) instructions for use.

The present disclosure further provides a kit comprising:
a) a flexible magnetic film;
b) aggrecan precursors chosen from the list comprising hyaluronic acid, glucosamine sulfate and/or chondroitin sulfate;
c) a dressing with at least one self-adhesive surface wherein the dressing increases the proprioception of the subject;
d) instructions for use.

### General

Those skilled in the art will appreciate that the disclosure described herein is susceptible to variations and modifications other than those specifically described. It is to be understood that the disclosure includes all such variations and modifications. The disclosure also includes all of the steps, features, compositions and compounds referred to or indicated in the specification, individually or collectively and any and all combinations or any two or more of the steps or features.

The present disclosure is not to be limited in scope by the specific embodiments described herein, which are intended for the purpose of exemplification only. Functionally equivalent products, compositions and methods are clearly within the scope of the disclosure as described herein.

As used herein the term "derived" and "derived from" shall be taken to indicate that a specific integer may be obtained from a particular source albeit not necessarily directly from that source.

As used herein, the singular forms "a," "an" and "the" include plural references unless the context clearly dictates otherwise.

Throughout this specification, unless the context requires otherwise, the word "comprise", or variations such as "comprises" or "comprising", will be understood to imply the inclusion of a stated integer or group of integers but not the exclusion of any other integer or group of integers.

Other than in the operating example, or where otherwise indicated, all numbers expressing quantities of ingredients, reaction conditions, and so forth used in the specification and claims are to be understood as being modified in all instances by the term "about". Accordingly, unless indicated to the contrary, the numerical parameters set forth in the specification and claims are approximations that may vary depending upon the desired properties sought to be obtained by the present disclosure. Hence "about 80 %" means "about 80 %" and also "80 %". At the very least, each numerical parameter should be construed in light of the number of significant digits and ordinary rounding approaches.

Notwithstanding that the numerical ranges and parameters setting forth the broad scope of the disclosure are approximations, the numerical values set forth in the specific examples are reported as precisely as possible. Any numerical value; however, inherently contains certain errors necessarily resulting from the standard deviation found in their respective testing measurements

Other definitions for selected terms used herein may be found within the detailed description of the disclosure and apply throughout. Unless otherwise defined, all other scientific and technical terms used herein have the same meaning as commonly understood to one of ordinary skill in the art to which the disclosure belongs.

The following examples serve to more fully describe the manner of using the above-described disclosure, as well as to set forth the best modes contemplated for carrying out various aspects of the disclosure. It is understood that these methods in no way serve to limit the true scope of this invention, but rather are presented for illustrative purposes.

### Examples

Further features of the present disclosure are more fully described in the following non-limiting Examples. This description is included solely for the purposes of exemplifying the present disclosure. It should not be understood as a restriction on the broad description of the disclosure as set out above.

### Example 1

### Patch

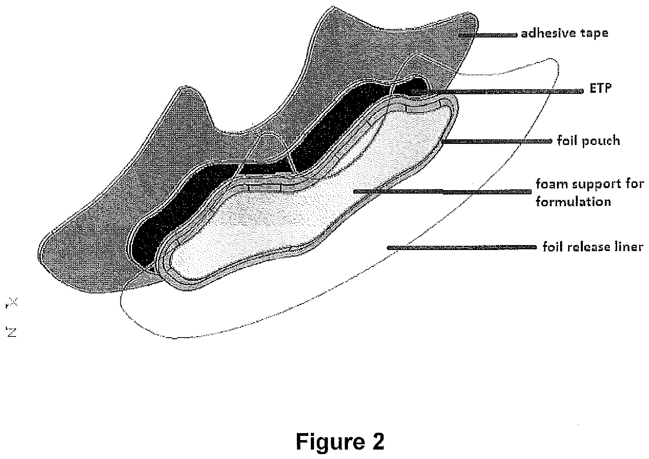

The patch of Figure 2 is designed to firmly place the active gel composition comprising hyaluronic acid, glucosamine sulfate and chondroitin sulfate against the skin with a flexible magnetic film (ETP) behind to accelerate transdermal migration of the aggrecan precursors.

The adhesive part of the patch is four-way stretchable woven spandex-like elastomer backing tape, shaped to support the joint (for example knee) and provide proprioceptive properties; and to secure the active gel against the skin.

The active gel is housed in a foam/silicon gel scaffold in an aluminium foil module, attached to a shaped sheet of flexible magnetic film (ETP), which is in turn adhered to the backing tape. The active gel in the module is protected with an aluminium foil release liner that, on removal, acts to expose the surface of the active gel in the module, ready for application to the skin. The aluminium foil release liner may also be large enough to cover substantially the entire tape and thus removal of the release liner will further expose the backing tape adhesive that will secure the entire device to the target area.

Of particular importance is the ability to house the active gel in a module that allows for ease of application with no leakage and no visible residue after use. The active gel compartment may be permanently affixed to the backing tape of the patch, or it may be removable. The foam and/or silicon scaffold in the module is also replaceable with alternative scaffolds to hold the active gel, or can be removed entirely such that the active gel is provided alone in an aluminium foil module.

The patch design allows for deliberate changes to be made to the type and shape of the stretchable fabric, the shape, magnetic strength, density and directionality of the flexible magnetic film ETP strip, the housing of the gel and the nature of the release mechanism and how the product is applied to the skin.

### Active gel compositions for topical delivery of aggrecan precursors

**Table 1: Example Composition**

| **Ingredient Purpose** | **Component** | **Conc (% by weight composition)** | **Other Options** |
|---|---|---|---|
| Active | Glucosamine sulfate | 1 | |
| Active | Hyaluronic acid | 0.25 | |
| Active | Chondroitin sulfate | 0.25 | |
| Sensate | Menthol | 0.1-4 | Menthyl lactate, camphor, eucalyptus oil, essential oils |
| Sensate | Thymol | 0.1 | Menthyl lactate, camphor, eucalyptus oil, essential oils |
| Solvent | Propylene Glycol | 30 | Butvlene glycol, various glycol esters, |
| Solvent | Ethanol | 21 | Isopropanol |
| Preservative | Germaben | 1 | Parabens, imidureas, Kathon |
| Viscosity agent | Gelatin | 10 | Carboxymethyl cellulose, carbopols, hydroxyethyl cellulose |
| Anti-oxidant | Vitamin C | 0.1 | Sodium ascorbyl phosphate |
| Anti-oxidant | Vitamin E | 0.5 | Tocopheryl acetate, BHT, BHA |
| Solvent / Manufacturing aid | Water | 31.7 | |
| Colour | Brilliant Blue #1 CI42090 | 0.1 | All other approved colours |

### Example 2

### Skin Penetration of Composition

*Ex vivo* Franz cell skin penetration studies on separated human skin were conducted in the OBJ laboratories to assess the accelerating effect of various flexible magnetic film ETP magnetic fields on the composition of the present disclosure comprising glucosamine, chondroitin and hyaluronic acid.

A gel composition as provided in Table 2 was tested for magnetic field assisted penetration versus (i) passive penetration and (ii) the market leading topical cream, Jointace® (containing topical glucosamine and chondroitin).

**Table 2: Example Composition**

| **Ingredient** | **Cone %** |
|---|---|
| Glucosamine sulfate | 1 |
| Hyaluronic acid | 0.25 |
| Chondroitin sulfate | 0.25 |
| Menthol | 0.1-4 |
| Thymol | 0.1 |
| Propylene Glycol | 30 |
| Ethanol | 21 |
| Germaben | 1 |
| Gelatin | 10 |
| Vitamin C | 0.1 |
| Vitamin E | 0.5 |
| Water | 31.7 |
| Brilliant Blue #1 CI42090 | 0.1 |

A flexible magnetic film microarray, comprising a repeating diamond arrangement of magnetic gradients, as illustrated in Figure 1, comprising a neutral, -25mT and +25mT flux gradient, disbursed at a distance of 5mm between centres, was found to deliver 14 times more glucosamine from our test gel than Jointace® after 8 hours. Even after 60 min, more than 2.7 times the glucosamine concentration was delivered through excised epidermal tissues in a Franz type diffusion study.

A modified flexible magnetic film microarray comprising a repeating diamond arrangement of magnetic gradients, as illustrated in Figure 1, comprising a neutral zone of 10mT with flux gradients of -25mT and +12mT, disbursed at a distance of 10 mm between centres delivered over 5 times more chondroitin than Jointace® after only 280 min.

A further modified flexible magnetic film microarray comprising a repeating diamond arrangement of magnetic gradients, as illustrated in Figure 1, comprising a neutral zone of 12mT with flux gradients of -10mT and +25mT, disbursed at a distance of 6 mm between centres was found to deliver twice as much hyaluronic acid versus passive diffusion after 24 hours. Jointace® does not contain hyaluronic acid and was not tested.

The results show that the combination of the composition of the present disclosure with the flexible magnetic film of the present disclosure was able to deliver significantly high levels of the three aggrecan precursors through excised human skin in the Franz cell experiments than either passive diffusion or Jointace® joint cream.

### Example 3

An Aggregated Locomotor Function (ALF) study was employed to assess the capability of a knee patch used topically over 2 weeks and equipped with a flexible magnetic film and a topical gel containing a mixture of pro-aggrecans (glucosamine, chondroitin, hyaluronic acid) to improve knee function and pain scores amongst a cohort of physically active males with self-described knee joint problems.

The ALF study combined 6 knee intensive challenges (3 timed studies and 3 distance/strength studies) and was developed to evaluate the combined effect of the patch technology with the pro-aggrecan gel of the present disclosure applied on the skin surface just under the patella.

### • Study Design

An open label test/re-test study into the efficacy of the present disclosure applied in the form of a wearable knee patch, in the enhanced delivery of the formulation stated in Table 2 was undertaken. The study evaluated improved lower limb function and perception in adult males 35-55 using an Aggregated Locomotor Function (ALF) score (McCarthy and Oldham, Rheumatology, 43, 514-7 (2004)). The magnetic microarray used in this case was a repeating diamond arrangement of magnetic gradients, as illustrated in Figure 1, comprising a neutral zone of 12mT with flux gradients of -10mT and +25mT, disbursed at a distance of 6 mm.

The ALF score is a simple measure of observed locomotor function using timed walking, stairs and transfers. The ALF takes approx. 10-15 min to administer and has reliable inter-tester repeatability and is very responsive to evaluation of test outcomes. McCarthy and Oldham (2004) showed that the ALF, when applied to locomotor tests in patients with OA of the knee, exhibited intra-class correlation coefficient (ICC) statistics of ICC 2k 0.99; 95% CI 0.98-0.99 and low SEM (0.86s).

Seventeen males subjects were recruited for the present test.

**Table 3: Study subject criteria**

| **Inclusion Criteria** | **Exclusion Criteria:** |
|---|---|
| Males aged 35-55 | No patello-femoral joint dysfunction |
| Prior history of knee injury requiring arthroscopic surgery and/or; Prolonged history of recurrent knee pain | No ligamentous deficiency |
| In good general health | History of cardiac disease; high blood pressure; asthma; diabetes |
| Currently participating in regular physical activity (>2hr/week) | |

Testing was carried out at baseline and after two weeks of knee guard use.

### Tests:

- Timed up and down stairs
- Timed step test
- Timed balsom agility run
- Measured vertical jump
- Measured hop for distance
- Measured drop jump double hop

### • Results

In the study, 94% of subjects were found to have overall improved knee functionality vs. baseline after two weeks of product use.

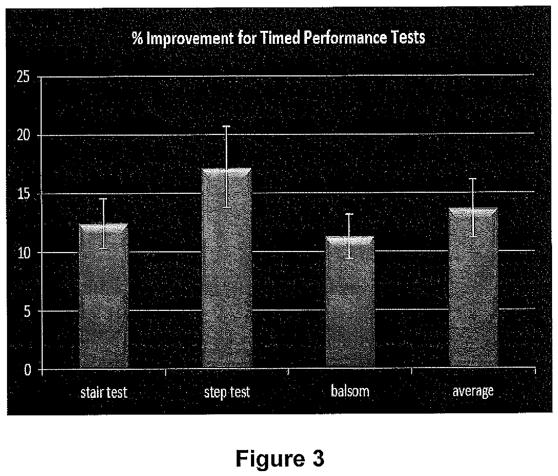

Improvement in performance in the timed challenges was found in 16 of 17 participants while the overall measured average percentage improvement in the timed performance tests was 14% compared with baseline 2 weeks previously. The Stair test, Step Test and Balsom Agility Test were used to assess timed performance. The 14% overall improvement represented a range of significant decreased performance times between 1.38 and 3.61 seconds vs. baseline. The Wall Squat proved to be highly variable in execution and poorly repeatable mainly due to the highly variable athletic ability of the participants (Table 4; Figure 3).

**Table 4: Results**

| **Test** | **Baseline (Av sec)** n=17 | **After 2 Weeks Product Use (Av sec)** n=17 | **% Difference Improvement** | **P Paired t-Test** |
|---|---|---|---|---|
| Timed Stair | 11.03 | 9.66 | 12.49 | 9.06x10⁻⁵ |
| Timed Step | 20.96 | 17.30 | 17.22 | 7.09x10⁻⁴ |
| Balsom | 13.81 | 12.25 | 11.29 | 3.59x10⁻⁵ |
| | | | | |
| Accumulated Timed Data per Panelist | 45.80 | 39.26 | 14.00 | 3.60x10⁻⁵ |
| | | | | |
| OVERALL % IMPROVEMENT | | | 14 | |

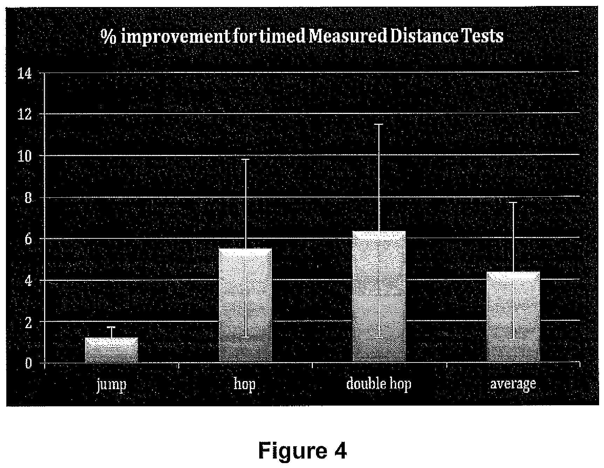

Improvement in the distance challenges was found in 12 of 17 participants with a directional improvement of 4.4% in performance in the more challenging distance/strength challenges. The Vertical Jump Test, Single Leg Hop and Double Jump Hop tests were used to measure performance. The result is likely more reflective of improved technique and test familiarity with a directional improvement in joint strength and mobility (Table 5, Figure 4).

**Table 5: Results**

| **Test** | **Baseline (ave cm)** n=17 | **After 2 Weeks Product Use (ave cm)** n=17 | **% Difference Improvement** | **P Paired t-Test** |
|---|---|---|---|---|
| Vertical Jump | 258 | 262 | 1.28 | 0.015 |
| Single Leg Hop | 104 | 109 | 5.54 | 0.144 |
| Double Jump Hop | 246 | 261 | 6.34 | 0.144 |
| OVERALL % IMPROVEMENT | | | 4.4 | |

Overall improvement in timed tests was found in 94% (16 of 17) of test subjects while only 11 of 17 test subjects were found to improve in the strength / agility tests. This further reinforces the finding that the use of the test products influenced measurable significant improvements in timed activities while strength and agility testing required either a longer period of use or a more controlled, higher statistically powered testing regime.

Aggregated voluntary comments from the test diaries indicated an overall favourable rating for product performance of 67% vs. an unfavourable rating of 33%. Overall including all comments the fav/unfav rating was 56/44. The favourable comments were driven by "made knee feel better", "pain reduction", "felt better", "felt good when running", "more mobility", "comfortable to use", "forgot I was wearing it". Unfavourable comments were driven by "sticky surface", "not much improvement" and "burning sensation", "skin irritation".

**Table 6: Voluntary user comments**

| **Favourable Comments** | **n** | **Unfavourable Comments** | **n** |
|---|---|---|---|
| Don't feel the patch, comfortable, forgot I was wearing it | 9 | Broken patch, oozed liquid | 7 |
| Felt good running/cycling/regular exercise, less painful, more mobility | 11 | Sticky surface no good, came loose | 13 |
| Pain free | 6 | Sweaty skin, hairy leg, cause problems | 5 |
| Made knee feel better, pain reduction, noticed improvement | 25 | Not much improvement, same as before | 25 |
| Amazing, felt good | 4 | Gel needs a different colour - green to skin tone | 1 |
| Noticeable difference | 2 | Burning skin sensation, discomfort, skin irritated | 11 |
| No complications wearing | 7 | | |
| Soothing, cooling | 6 | | |
| Nice warmth on application | 2 | | |
| Not messy | 1 | | |
| Made a difference | 2 | | |
| Really happy with patch | 1 | | |
| Feel fluid feeding the knee | 1 | | |
| Not as much joint grinding sound as before | 3 | | |

**Table 7: Collated user comments**

| | **Favourable (%)** | **Unfavourable (%)** |
|---|---|---|
| Overall | 56 | 44 |
| Performance | 67 | 33 |
| Comfort/Application | 42 | 58 |
| In use feel | 45 | 55 |

This study was able to show that daily use of a knee guard patch for 2 weeks by active sports people resulted in a significant improvement in accumulated timed performance tests. A directional (not significant in this study) improvement was found after 2 weeks in the combined strength and agility tests.

### Example 4

### Enhanced Knee Function Of Active Males Using Enhanced Musculoskeletal Patches

### Method

Active males aged 35-55 who experience regular knee pain were recruited after completing an online questionnaire. All registrations were vetted for their suitability to the selection criteria. 20 males were selected for participation with 17 successfully returning for the re-test, thereby making their overall results valid.

An Aggregated Locomotor Function (ALF) protocol was employed in combination with the musculoskeletal knee patch used topically over 2 weeks. The patch was equipped with a magnetic microarray and a topical gel containing a mixture of pro-aggrecan ingredients (glucosamine, chondroitin, hyaluronic acid) in order to assess knee function and pain scores amongst a cohort of physically active males with self-described knee joint problems.

The topical gel had the following composition:

| **Component** | **Conc (% by weight composition)** |
|---|---|
| Glucosamine sulfate | 1 |
| Hyaluronic acid | 0.25 |
| Chondroitin sulfate | 0.25 |
| Menthol | 0.1-4 |
| Thymol | 0.1 |
| Propylene Glycol | 30 |
| Ethanol | 21 |
| Germaben | 1 |
| Gelatin | 10 |
| Vitamin C | 0.1 |
| Vitamin E | 0.5 |
| Water | 31.7 |
| Brilliant Blue #1 CI42090 | 0.1 |

The magnetic microarray was a 25p6 linear array, comprising 25 poles per inch, 0.6mm thickness and with a peak field strength of 172 Gauss.

An open label test re-test study was made of the efficacy of the musculoskeletal patch in improving lower limb function and perception in adult males 35-55 using the (ALF) score [C.J. McCarthy and J.A. Oldham, Rheumatology, 43, 514-7 (2004)]. The ALF score is a simple measure of observed locomotor function using timed walking, stairs and transfers. The ALF takes approx. 10-15 min to administer and has reliable inter-tester repeatability and is very responsive to evaluation of test outcomes. In another study, the ALF in patients with OA of the knee exhibited intra-class correlation coefficient (ICC) statistics of ICC 2k 0.99; 95% CI 0.98-0.99 and low SEM (0.86s) [C.J. McCarthy and J.A. Oldham, Rheumatology, 43, 514-7 (2004)]. Participants also kept personal diaries to record daily changes in their pain levels and mobility. ALF scores were measured before and after patch usage to determine overall changes in performance results, with the comparisons made using Paired t-Test analysis.

The ALF study combined 6 knee intensive challenges (3 timed studies and 3 distance/strength studies) and was developed to evaluate the combined effect of the patch technology with TGA approved lubricating pro-aggrecan gel applied on the skin surface just under the patella.

| **Subjects:** | 17 males |
|---|---|
| **Inclusion Criteria:** | Males aged 35-55 |
| | Prior history of knee injury requiring arthroscopic surgery and/or; |
| | Prolonged history of recurrent knee pain |
| | In good general health |
| | Currently participating in regular physical activity (>2hr/week) |
| **Exclusion Criteria:** | No patello-femoral joint dysfunction |
| | No ligamentous deficiency |
| | No history of cardiac disease |
| | No history of high blood pressure |
| | No history of asthma |
| | No history of diabetes |
| **Measures:** | Testing carried out at baseline and at two weeks of |
| | musculoskeletal patch usage |
| **Tests:** | Timed up and down stairs |
| | Timed step test |
| | Timed Balsom agility run |
| | Measured vertical jump |
| | Measured hop for distance |
| | Measured drop jump double hop |

### Results

The average percentage improvement in the timed performance tests was 14%.

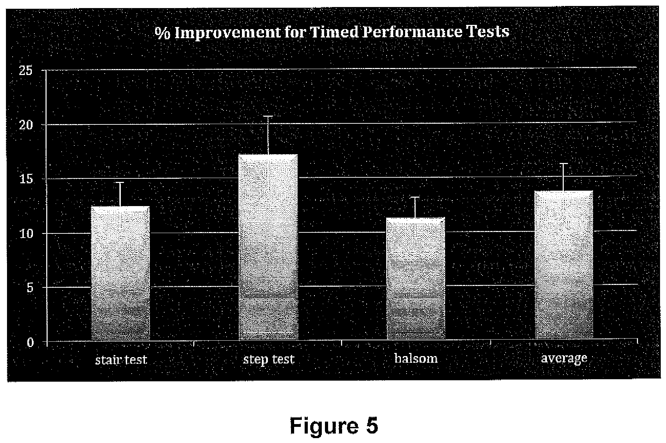

The Stair test, Step Test and Balsom Agility Test were used to assess timed performance. The 14% overall improvement represented a range of significant decreased performance times between 1.38 and 3.61 seconds vs. baseline (Table 8, Figure 5). The Wall Squat proved to be highly variable in execution and poorly repeatable mainly due to the highly variable athletic ability of the participants.

**Table 8: Results**

| **Test** | **Baseline (Av Sec) N=17** | **After 2 Weeks Product Use (Av Sec) N=17** | **Difference Improvement (%)** | **Paired t-Test** |
|---|---|---|---|---|
| Timed Stair | 11.03 | 9.66 | 12.49 | 9.06x10⁻⁵ |
| Timed Step | 20.96 | 17.30 | 17.22 | 7.09x10⁻⁴ |
| Balsom | 13.81 | 12.25 | 11.29 | 3.59x10⁻⁵ |
| Accumulated Timed Data per Panelist | 45.80 | 39.26 | 14.00 | 3.60x10⁻⁵ |
| **OVERALL IMPROVEMENT** | | | **14%** | |

The average percentage improvement in measured distance tests was 4.4%.

The Vertical Jump Test, Single Leg Hop and Double Jump Hop tests were used to measure performance (Table 9). The result is likely more reflective of improved technique and test familiarity with a directional improvement in joint strength and mobility. The measured difference was not significant overall.

**Table 9: Results**

| **Test** | **Baseline (Av Sec) N=17** | **After 2 Weeks Product Use (Av Cm) N=17** | **Difference Improvement (%)** | **Paired t-Test** |
|---|---|---|---|---|
| Vertical Jump | 258 | 262 | 1.28 | 0.015 |
| Single Hop | 104 | 109 | 5.54 | 0.144 |
| Double Hop | 246 | 261 | 6.34 | 0.144 |
| **OVERALL IMPROVEMENT** | | | **4.4%** | |

Overall improvement in timed tests was found in 94% (16 of 17) of test subjects while only 11 of 17 test subjects were found to improve in the strength / agility tests.

This further reinforces the finding that the use of the test products influenced measurable significant improvements in timed activities while strength and agility testing required either a longer period of use or a more controlled, higher statistically powered testing regime.

Aggregated voluntary comments from the test diaries indicated a 64% (11 of 17) overall favourable rating for reduction in pain and increased mobility

Unsolicited voluntary comments from the daily diaries indicated 11 of the 17 participants recorded a noticeable reduction in their knee pain at some point over the 2-week trial. Comments relating to feeling 'more confident' and 'increased mobility' were also accepted as an indication that the participant was experiencing less pain (Table 10).

**Table 10: User Comments**

| **Favorable Comments** | **N** | **Unfavorable Comments** | **N** |
|---|---|---|---|
| Don't feel the patch, comfortable, forgot I was wearing it | 9 | Broken patch, oozed liquid | 7 |
| Felt good running/cycling/regular exercise, less painful, more mobility | 11 | Sticky surface no good, came loose | 13 |
| Pain free | 6 | Sweaty skin, hairy leg, cause problems | 5 |
| Made knee feel better, pain reduction, noticed improvement | 25 | Not much improvement, same as before | 25 |
| Amazing, felt good | 4 | Gel needs a different colour-green to skin tone | 1 |
| Noticeable difference | 2 | Burning skin sensation, discomfort, skin irritated | 11 |
| No complications wearing | 7 | | |
| Soothing, cooling | 6 | | |
| Nice warmth on application | 2 | | |
| Not messy | 1 | | |
| Made a difference | 2 | | |
| Really happy with patch | 1 | | |
| Feel fluid feeding the knee | 1 | | |
| Not as much joint grinding sound as before | 3 | | |

In summary, seventeen participants successfully completed the study with 94% found to have an overall improvement in knee functionality. Improvement in performances of timed challenges was found in 16 of 17 participants with an overall average improvement of 14% compared with baseline (3.60x10⁻⁵). Improvement in the distance challenges was found in 12 of 17 participants indicating a directional improvement of 4.4%. An overall rating of 64% was attributed to positive voluntary comment relating to pain reduction, more mobility and feeling better.

### Example 5

### Franz diffusion cells studies

Diffusion studies were conducted using standard Franz diffusion cells with an approximate receptor volume of 3 mL and a membrane area of approximately 1cm². 0.1 mL of either Jointace® or Bodyguard formulation according to the present disclosure was applied to a membrane consisting of excised human epidermis. The excised human epidermis was treated with either Bodyguard formulation in conjunction with a magnetic-microarray (ETP film) by suspending the magnetic microarray directly above the skin surface; or Jointace®. The receptor fluid was approximately 3ml of pure water. Diffusion studies were conducted over 24 hours with readings at intervals of 0, 30, 60, 120, 240, 360 and 1440 mins.

The topical Bodyguard gel had the following composition:

| **Component** | **Conc (% by weight composition)** |
|---|---|
| Glucosamine sulfate | 1 |
| Hyaluronic acid | 0.25 |
| Chondroitin sulfate | 0.25 |
| Menthol | 0.1-4 |
| Thymol | 0.1 |
| Propylene Glycol | 30 |
| Ethanol | 21 |
| Germaben | 1 |
| Gelatin | 10 |
| Vitamin C | 0.1 |
| Vitamin E | 0.5 |
| Water | 31.7 |
| Brilliant Blue #1 CI42090 | 0.1 |

The magnetic microarray ETP films used were:
- 25p6 linear array comprising 25 poles per inch, 0.6mm thickness and a peak field strength of 172 Gauss;
- 17p6 linear array comprising 17 poles per inch, 0.6mm thickness and a peak field strength of 292 Gauss;
- 12p6 x 25p2 array comprising a repeating diamond arrangement of magnetic gradients, comprising a neutral, -27mT and +27mT flux gradient, disbursed at a distance of 3mm between centres [the 25p6 linear array comprises 25 poles per inch and a peak field strength of 172 Gauss; the 12p6 linear microarray comprises 12 poles per inch and a peak field strength of 260 Gauss, the two arrays are offset at 90° and the film is 0.8mm in thickness].

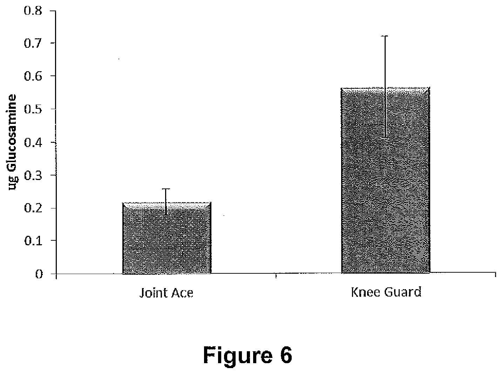

Glucosamine levels in each sample were determined using a validated HPLC method. See Figure 6.

**Table 11: Penetration of glucosamine through excised human epidermis as determined from Franz cell diffusion studies**

| | **Jointace** | **Passive** | **ETP 25p6** | **ETP 17p6** | **ETP 12p6 x 25p2** |
|---|---|---|---|---|---|
| **0** | 0.000 | 0.605 | 0.000 | 0.000 | 0.675 |
| **30** | 0.233 | 0.735 | 0.042 | 0.659 | 0.652 |
| **60** | 0.382 | 0.607 | 2.244 | 1.252 | 0.696 |
| **120** | 0.692 | 1.009 | 10.769 | 5.355 | 0.968 |
| **240** | 0.900 | 4.658 | 28.745 | 14.594 | 6.585 |
| **360** | 1.614 | 8.689 | 50.296 | 27.472 | 12.210 |
| **1440** | 4.651 | 46.356 | 152.405 | 106.492 | 56.391 |

| | | | | | |
|---|---|---|---|---|---|
| Data shown is average penetration in ug/cm2 | | | | | |

### Tape Stripping

The subject's volar forearm or upper arm was delineated into two circular regions (2.2cm in diameter) which were designated "Jointace" and "Bodyguard". An aliquot of proprietary Jointace® formulation was applied to the Jointace region while an aliquot of the Bodyguard formulation was was applied to the Bodyguard region. The BodyGuard region was then covered with an ETP magnetic patch.

The topical Bodyguard gel had the following composition:

| **Component** | **Conc (% by weight composition)** |
|---|---|
| Glucosamine sulfate | 1 |
| Hyaluronic acid | 0.25 |
| Chondroitin sulfate | 0.25 |
| Menthol | 0.1-4 |
| Thymol | 0.1 |
| Propylene Glycol | 30 |
| Ethanol | 21 |
| Germaben | 1 |
| Gelatin | 10 |
| Vitamin C | 0.1 |
| Vitamin E | 0.5 |
| Water | 31.7 |
| Brilliant Blue #1 CI42090 | 0.1 |

The magnetic microarray was a 25p6 array, comprising 25 poles per inch, 0.6mm thickness and had a peak field strength of 172 Gauss.

After an application period of two hours the excess donor liquid or patch was removed from each region with tissue paper and then tape stripped using the adapted procedure of Lademann *et al* (J. Lademann, U. Jacobi, C. Surber, H.-J. Weigmann, J.W. Fluhr. The tape stripping procedure - evaluation of some critical parameters. European Journal of Pharmaceutics and Biopharmaceutics 72 (2009) 317-323).

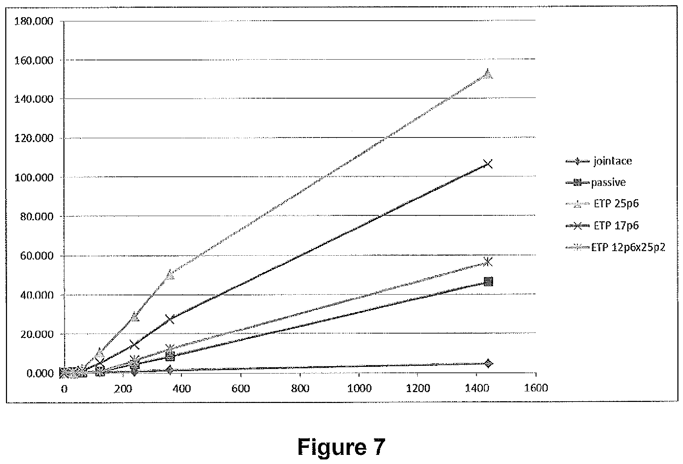

The average enhancement of penetration by co-administration of the Bodyguard formulation and the ETP patch was 2.6 times. See Figure 7.

**Table 12: Loading of Glucosamine (ug) in the Stratum Corneum determined by tape stripping**

| **Subject** | **Jointace®** | **Bodyguard** |
|---|---|---|
| **1** | 0.193309475 | 0.543970463 |
| **2** | 0.204911101 | 0.401088929 |
| **3** | 0.369463682 | 1.160887603 |
| **4** | 0.157214619 | 0.33091433 |
| **5** | 0.160704581 | 0.382392158 |
| **average** | **0.217120692** | **0.563850697** |
| **st dev** | 0.087601298 | 0.342974575 |
| **sem** | **0.039176492** | **0.153382893** |

Numerous variations and modifications of the above-described modes of carrying out the various embodiments of this disclosure will be apparent to those skilled in the art, based on the above teachings related to the disclosed disclosure, without departing from the basic inventive concepts. The above embodiments of the disclosure are merely exemplary and should not be construed to be in any way limiting and all such variations and modifications are to be considered within the scope of the present disclosure, the nature of which is to be determined from the foregoing description.

## Claims

1. A flexible magnetic film for use in treating joint pain or degeneration in a subject, wherein the film improves delivery of aggrecan precursors to a subject in need thereof, wherein the film is applied to the skin of the subject on a joint that has joint pain or degeneration, and wherein a composition comprising at least two aggrecan precursors chosen from the list consisting of hyaluronic acid, glucosamine sulfate and/or chondroitin sulfate is applied between the skin and the flexible magnetic film, and wherein at least part of the of the flexible magnetic film and composition are covered with a dressing, wherein the dressing increases the proprioception of the subject.

2. The flexible magnetic film for use according to claim 1, wherein application of the film increases the penetration of the aggrecan precursors through the skin of the subject.

3. The flexible magnetic film for use according to claim 2, wherein the increased penetration of the aggrecan precursors through the skin of the subject is by:
(a) increased movement of the aggrecan precursors from the composition towards the skin of the subject; and/or
(b) temporarily altering the permeability of the skin of the subject, optionally wherein the increased movement of the aggrecan precursors is via diamagnetic repulsion.

4. A device for use in the treatment or prevention of joint pain and degeneration in a subject in need thereof, wherein the device comprises:
a) a composition comprising at least two aggrecan precursors chosen from the list consisting of hyaluronic acid, glucosamine sulfate and/or chondroitin sulfate; and
b) a flexible magnetic film; and,
c) a dressing, wherein the dressing increases the proprioception of the subject, wherein the dressing covers at least part of the flexible magnetic film and composition;
wherein the composition is applied between the skin of the subject and the flexible magnetic film, wherein the film is applied on a joint that has joint pain or degeneration.

5. A kit for use in the delivery of exogenous aggrecan precursors to a joint of a subject that has joint pain or degeneration, wherein the kit comprises:
a) a flexible magnetic film comprising at least one partially or completely self-adhesive surface;
b) at least two aggrecan precursors chosen from the list comprising hyaluronic acid, glucosamine sulfate and/or chondroitin sulfate;
c) a dressing with at least one self-adhesive surface; and,
d) instructions for use.

6. The kit according to claim 5, wherein the dressing increases the proprioception of the subject.

7. The flexible magnetic film for use according to any one of claims 1 to 3, device for use according to claim 4, or kit according to claim 5 or 6, wherein the composition comprises one of the following combinations: hyaluronic acid and glucosamine sulfate; hyaluronic acid and chondroitin sulfate; glucosamine sulfate and chondroitin sulfate; hyaluronic acid, glucosamine sulfate and chondroitin sulfate.

8. The flexible magnetic film for use according to any one of claims 1 to 3, device for use according to claim 4, or kit according to claim 5 or 6, wherein the subject is suffering from osteoarthritis.

9. The flexible magnetic film for use according to any one of claims 1 to 3, device for use according to claim 4, or kit according to claim 5 or 6, wherein the composition further comprises a sensate ingredient.

10. The flexible magnetic film for use according to any one of claims 1 to 3, device according to claim 4, or kit according to claim 5 or 6, wherein the aggrecan precursors are present in individual amounts of from about 0.001 to about 5% by weight of the composition.

11. The flexible magnetic film for use according to any one of claims 1 to 3, device for use according to claim 4, or kit according to claim 5 or 6, wherein the magnetic elements of the flexible magnetic film have the following parameters:
i) a horizontal off-set of between about 1 and 10 millimetres, 2 and 8 millimetres, or 3 and 7 millimetres; and/or
ii) a repetition rate of about 1 and 10 elements per centimetre, 1 and 6 elements per centimetre or 1.5 and 4 elements per centimetre; and/or
iii) the poles of the magnetic elements in a particular spatial region are between about 1.0 mm to 10mm apart, or 1.0 mm to 5.0mm apart; and/or
iv) the magnetic flux of each magnetic pole of the magnetic elements is between about 1mT and 100mT, 1mT to about 60mT, or 12mT to 45mT; and/or
v) the delta flux between the magnetic flux of two adjacent poles of opposite polarity is between about 2mT and 200mT, 20mT to 140mT, or 20mT to 90mT.

12. The flexible magnetic film for use, device for use, or kit according to claim 11 wherein the magnetic elements of the flexible magnetic film have a linear arrangement.

13. The flexible magnetic film for use, device for use, or kit according to claim 12 wherein the magnetic elements of the flexible magnetic film have the following parameters: between 5 and 10 poles per cm and a magnetic flux of between 150 Gauss and 200 Gauss, optionally wherein the magnetic elements of the flexible magnetic film have the following parameters: 10 poles per cm and a magnetic flux of 172 Gauss.

## Patentansprüche

1. Flexible magnetische Folie zur Verwendung bei einer Behandlung von Gelenkschmerzen oder -degeneration bei einem Subjekt, wobei die Folie ein Abgeben von Aggrecan-Vorläufern an ein Subjekt, das dies benötigt, verbessert, wobei die Folie auf die Haut des Subjekts auf einem Gelenk aufgebracht wird, das Gelenkschmerzen oder -degeneration aufweist, und wobei eine Zusammensetzung, die wenigstens zwei Aggrecan-Vorläufer umfasst, die aus der Liste ausgewählt sind, die aus Hyaluronsäure, Glucosaminsulfat und/oder Chondroitinsulfat besteht, zwischen der Haut und der flexiblen magnetischen Folie aufgebracht wird, und wobei wenigstens ein Teil der flexiblen magnetischen Folie und der Zusammensetzung mit einem Verband bedeckt sind, wobei der Verband die Propriozeption des Subjekts steigert.

2. Flexible magnetische Folie zur Verwendung nach Anspruch 1, wobei das Aufbringen der Folie das Eindringen der Aggrecan-Vorläufer durch die Haut des Subjekts steigert.

3. Flexible magnetische Folie zur Verwendung nach Anspruch 2, wobei das gesteigerte Eindringen der Aggrecan-Vorläufer durch die Haut des Subjekts durch Folgendes erfolgt:
(a) gesteigerte Bewegung der Aggrecan-Vorläufer von der Zusammensetzung zu der Haut des Subjekts; und/oder
(b) vorübergehendes Verändern der Durchlässigkeit der Haut des Subjekts, optional wobei die gesteigerte Bewegung der Aggrecan-Vorläufer über eine diamagnetische Abstoßung erfolgt.

4. Vorrichtung zur Verwendung bei der Behandlung oder Vorbeugung von Gelenkschmerzen und -degeneration bei einem Subjekt, das diese benötigt, wobei die Vorrichtung Folgendes umfasst:
a) eine Zusammensetzung, die wenigstens zwei Aggrecan-Vorläufer umfasst, die aus der Liste ausgewählt sind, die aus Hyaluronsäure, Glucosaminsulfat und/oder Chondroitinsulfat besteht; und
b) eine flexible magnetische Folie; und
c) einen Verband, wobei der Verband die Propriozeption des Subjekts steigert, wobei der Verband wenigstens einen Teil der flexiblen magnetischen Folie und der Zusammensetzung bedeckt;
wobei die Zusammensetzung zwischen der Haut des Subjekts und der flexiblen magnetischen Folie aufgetragen wird, wobei die Folie auf ein Gelenk aufgetragen wird, das Gelenkschmerzen oder -degeneration aufweist.

5. Kit zur Verwendung bei dem Abgeben von exogenen Aggrecan-Vorläufern an ein Gelenk eines Subjekts, das Gelenkschmerzen oder -degeneration aufweist, wobei das Kit Folgendes umfasst:
a) eine flexible magnetische Folie, die wenigstens eine teilweise oder vollständig selbstklebende Oberfläche umfasst;
b) wenigstens zwei Aggrecan-Vorläufer, die aus der Liste ausgewählt sind, die Hyaluronsäure, Glucosaminsulfat und/oder Chondroitinsulfat umfasst;
c) einen Verband mit wenigstens einer selbstklebenden Oberfläche; und
d) Anweisungen zur Verwendung.

6. Kit nach Anspruch 5, wobei der Verband die Propriozeption des Subjekts steigert.

7. Flexible magnetische Folie zur Verwendung nach einem der Ansprüche 1 bis 3, Vorrichtung zur Verwendung nach Anspruch 4 oder Kit nach Anspruch 5 oder 6, wobei die Zusammensetzung eine der folgenden Kombinationen umfasst:
Hyaluronsäure und Glucosaminsulfat;
Hyaluronsäure und Chondroitinsulfat;
Glucosaminsulfat und Chondroitinsulfat;
Hyaluronsäure, Glucosaminsulfat und Chondroitinsulfat.

8. Flexible magnetische Folie zur Verwendung nach einem der Ansprüche 1 bis 3, Vorrichtung zur Verwendung nach Anspruch 4 oder Kit nach Anspruch 5 oder 6, wobei das Subjekt an Arthrose leidet.

9. Flexible magnetische Folie zur Verwendung nach einem der Ansprüche 1 bis 3, Vorrichtung zur Verwendung nach Anspruch 4 oder Kit nach Anspruch 5 oder 6, wobei die Zusammensetzung ferner einen sinnlich wahrnehmbaren Inhaltsstoff umfasst.

10. Flexible magnetische Folie zur Verwendung nach einem der Ansprüche 1 bis 3, Vorrichtung nach Anspruch 4 oder Kit nach Anspruch 5 oder 6, wobei die Aggrecan-Vorläufer in einzelnen Mengen von etwa 0,001 bis etwa 5 Gew.-% der Zusammensetzung vorhanden sind.

11. Flexible magnetische Folie zur Verwendung nach einem der Ansprüche 1 bis 3, Vorrichtung zur Verwendung nach Anspruch 4 oder Kit nach Anspruch 5 oder 6, wobei die magnetischen Elemente der flexiblen magnetischen Folie die folgenden Parameter aufweisen:
i) einen horizontalen Versatz zwischen etwa 1 und 10 Millimetern, 2 und 8 Millimetern oder 3 und 7 Millimetern; und/oder
ii) eine Wiederholungsrate von etwa 1 und 10 Elementen pro Zentimeter, 1 und 6 Elementen pro Zentimeter oder 1,5 und 4 Elementen pro Zentimeter; und/oder
iii) die Pole der magnetischen Elemente in einem bestimmten räumlichen Bereich sind zwischen etwa 1,0 mm bis 10 mm auseinander oder 1,0 mm bis 5,0 mm auseinander; und/oder
iv) der magnetische Fluss jedes magnetischen Pols der magnetischen Elemente ist zwischen etwa 1 mT und 100 mT, 1 mT bis etwa 60 mT oder 12 mT bis 45 mT; und/oder
v) der Deltafluss zwischen dem magnetischen Fluss zweier angrenzender Pole entgegengesetzter Polarität ist zwischen etwa 2 mT und 200 mT, 20 mT bis 140 mT oder 20 mT bis 90 mT.

12. Flexible magnetische Folie zur Verwendung, Vorrichtung zur Verwendung oder Kit nach Anspruch 11, wobei die magnetischen Elemente der flexiblen magnetischen Folie eine lineare Anordnung aufweisen.

13. Flexible magnetische Folie zur Verwendung, Vorrichtung zur Verwendung oder Kit nach Anspruch 12, wobei die magnetischen Elemente der flexiblen magnetischen Folie die folgenden Parameter aufweisen:
zwischen 5 und 10 Polen pro cm und einen magnetischen Fluss zwischen 150 Gauß und 200 Gauß, optional wobei die magnetischen Elemente der flexiblen magnetischen Folie die folgenden Parameter aufweisen:
10 Pole pro cm und einen magnetischen Fluss von 172 Gauß.

## Revendications

1. Film magnétique flexible destiné à être utilisé dans le traitement de la douleur ou de la dégénérescence articulaire chez un sujet, le film améliorant l'administration de précurseurs d'aggrécane à un sujet qui en a besoin, le film étant appliqué sur la peau du sujet sur une articulation qui présente une douleur ou une dégénérescence articulaire, et une composition comprenant au moins deux précurseurs d'aggrécane choisis dans la liste constituée d'acide hyaluronique, de sulfate de glucosamine et/ou de sulfate de chondroïtine étant appliquée entre la peau et le film magnétique flexible, et au moins une partie du film magnétique flexible et de la composition étant recouverts d'un pansement, le pansement augmentant la proprioception du sujet.

2. Film magnétique flexible à utiliser selon la revendication 1, l'application du film augmentant la pénétration des précurseurs d'aggrécane à travers la peau du sujet.

3. Film magnétique flexible à utiliser selon la revendication 2, la pénétration accrue des précurseurs d'aggrécane à travers la peau du sujet se produisant par :
(a) mouvement accru des précurseurs d'aggrécane de la composition vers la peau du sujet ; et/ou
(b) modification temporaire de la perméabilité de la peau du sujet, éventuellement le mouvement accru des précurseurs d'aggrécane se faisant par répulsion diamagnétique.

4. Dispositif destiné à être utilisé dans le traitement ou la prévention de la douleur et de la dégénérescence articulaires chez un sujet qui en a besoin, le dispositif comprenant :
a) une composition comprenant au moins deux précurseurs d'aggrécane choisis dans la liste constituée d'acide hyaluronique, de sulfate de glucosamine et/ou de sulfate de chondroïtine ; et
b) un film magnétique flexible ; et,
c) un pansement, le pansement augmentant la proprioception du sujet, le pansement recouvrant au moins une partie du film magnétique flexible et de la composition ;
la composition étant appliquée entre la peau du sujet et le film magnétique flexible, le film étant appliqué sur une articulation qui présente une douleur ou une dégénérescence articulaire.

5. Kit destiné à être utilisé dans l'administration de précurseurs d'aggrécane exogènes à une articulation d'un sujet qui présente une douleur ou une dégénérescence articulaire, le kit comprenant :
a) un film magnétique flexible comprenant au moins une surface partiellement ou totalement auto-adhésive ;
b) au moins deux précurseurs d'aggrécane choisis dans la liste comprenant de l'acide hyaluronique, du sulfate de glucosamine et/ou du sulfate de chondroïtine ;
c) un pansement avec au moins une surface auto-adhésive ; et,
d) un mode d'emploi.

6. Kit selon la revendication 5, le pansement augmentant la proprioception du sujet.

7. Film magnétique flexible à utiliser selon l'une quelconque des revendications 1 à 3, dispositif à utiliser selon la revendication 4, ou kit selon la revendication 5 ou 6, la composition comprenant l'une des combinaisons suivantes : acide hyaluronique et sulfate de glucosamine ; acide hyaluronique et sulfate de chondroïtine ; sulfate de glucosamine et sulfate de chondroïtine ; acide hyaluronique, sulfate de glucosamine et sulfate de chondroïtine.

8. Film magnétique flexible à utiliser selon l'une quelconque des revendications 1 à 3, dispositif à utiliser selon la revendication 4, ou kit selon la revendication 5 ou 6, le sujet souffrant d'arthrose.

9. Film magnétique flexible à utiliser selon l'une quelconque des revendications 1 à 3, dispositif à utiliser selon la revendication 4, ou kit selon la revendication 5 ou 6, la composition comprenant en outre un ingrédient sensoriel.

10. Film magnétique flexible à utiliser selon l'une quelconque des revendications 1 à 3, dispositif selon la revendication 4, ou kit selon la revendication 5 ou 6, les précurseurs d'aggrécane étant présents en quantités individuelles d'environ 0,001 à environ 5 % en poids de la composition.

11. Film magnétique flexible à utiliser selon l'une quelconque des revendications 1 à 3, dispositif à utiliser selon la revendication 4, ou kit selon la revendication 5 ou 6, les éléments magnétiques du film magnétique flexible ayant les paramètres suivants :
i) un décalage horizontal compris entre environ 1 et 10 millimètres, 2 et 8 millimètres, ou 3 et 7 millimètres ; et/ou
ii) un taux de répétition d'environ 1 et 10 éléments par centimètre, 1 et 6 éléments par centimètre ou 1,5 et 4 éléments par centimètre ; et/ou
iii) les pôles des éléments magnétiques dans une région spatiale particulière étant espacés d'environ 1,0 mm à 10 mm, ou de 1,0 mm à 5,0 mm ; et/ou
iv) le flux magnétique de chaque pôle magnétique des éléments magnétiques étant compris entre environ 1 mT et 100 mT, 1 mT à environ 60 mT, ou 12 mT à 45 mT ; et/ou
v) le flux delta entre le flux magnétique de deux pôles adjacents de polarité opposée étant compris entre environ 2 mT et 200 mT, 20 mT à 140 mT ou 20 mT à 90 mT.

12. Film magnétique flexible à utiliser, dispositif à utiliser ou kit selon la revendication 11, les éléments magnétiques du film magnétique flexible ayant une disposition linéaire.

13. Film magnétique flexible à utiliser, dispositif à utiliser ou kit selon la revendication 12, les éléments magnétiques du film magnétique flexible ayant les paramètres suivants : entre 5 et 10 pôles par cm et un flux magnétique compris entre 150 Gauss et 200 Gauss, éventuellement les éléments magnétiques du film magnétique flexible ayant les paramètres suivants : 10 pôles par cm et un flux magnétique de 172 Gauss.
